# EUROPEAN PATENT APPLICATION

(11) **EP 2 980 576 A1**
(43) Date of publication of application: **03.02.2016**
(21) Application number: 14179338.0
(22) Date of filing: 31.07.2014
(51) Int. Cl.: G01N 27/49, G01N 33/18, G01N 27/38, G01N 27/416

(54) **Electrochemical sensor system and sensing method**

(71) Applicant: Electrochemical Sensor Technological (EST) Limited, Castle Eden, Durham TS27 4SU (GB)
(72) Inventor: Marshall, Robert Neil, Castle Eden, Durham TS27 4SA (GB)
(74) Representative: Archer, Graham John

(57) **Abstract**

A sensor system for assessing a target species in a liquid medium is disclosed. The sensor system includes a sensor element which is inserted a sample of liquid medium to be tested. The sensor element has three or more electrodes and receives a predetermined potential from a potentiostat. This potential is determined as the potential associated with an electrochemical reaction characteristic in the liquid medium of a target species. The sensor system also includes an output to display data corresponding to the current passing through the liquid medium which is indicative of the concentration of the target species and resulting from the electrochemical reaction which occurs.

## Description

The invention relates to a sensor system for the identification and/or measurement of levels of target species in aqueous solution, and in particular for the identification and/or determination of levels of contaminant species such as nutrient species in the aqueous phase.

The invention is particularly directed to the provision of such a sensor system in a convenient portable/hand-held and a continuously operated form and/or adapted for use for the identification and/or measurement of levels of target species in water in a real (i.e. non-laboratory) and in particular external environment. The invention also relates to a method for the identification and/or determination of levels of target species in aqueous solution.

Sensors already exist for the determination of the presence of contaminant species in water, in particular nutrient and related species such as ammonia, ammonium, nitrates, nitrites and other ions, which are found in natural and industrial water.

The measurement of these species at the low and at high levels of concentrations that may be present in aqueous fluids is very important in a number of areas, including:
a) High purity boiler feed water supplies for power stations.
b) Drinking water production and maintenance of purity of supplies, including untreated and treated river waters for water extraction.
d) Waste water and sewage treatment processes.
e) In fermentation processes and biotechnology applications.
f) Steel work effluents and other trade water wastes.
f) The agricultural industry has strong requirements to measure levels of ammonia/ammonium ion and like contaminants. Here salt rich fertilisers and their resultant leaching into water bodies are a concern.
g) Furthermore animal slurries and routes of disposal are sources where ammonia/ammonium ion and like contaminants are present.
h) In the food industry analysis of beer, wine, milk and meat products analysis is carried out for ammonia/ammonium ion concentration levels.
i) Clinical analysis in the medical world relies upon ammonia/ammonium ion measurements when considering liver and kidney efficiencies in particular urea and urine relationships. Even levels as low as 5 mg ammonia per 100 ml of blood are toxic to humans.

Existing basic devices often employ potentiometric principles where a potential only is measured. The output from the ion selective electrode (ISE) is indirectly related to the log of the concentration of the species of interest, in the water sample. A common example of an ISE is a glass pH electrode. These glass and other types of ion exchange membrane electrodes are readily available from a number of suppliers at costs often several hundred pounds. However, ISEs in general have well known problems and limitations, such as poisoning and restrictions of use in certain environments.

Ion exchange membrane plastic type electrodes suffer badly from the effects of debris in the aqueous sample, surfactants, particulate material, etc., can cause deterioration of the sensors performance. ISE electrodes whatever the type and nature of the membrane must therefore be routinely cleaned, ideally after each measurement.

Calibration of all ISEs is also a time consuming and costly procedure. The procedure requires at the very least a minimum of laboratory training so that the electrode and measured value obtained can be relied upon. Hence care and a fair degree of scientific skill are necessary to ensure satisfactory operation.

A most important limitation of the present sensor technology is the inability of such sensors to be used directly in waters which have high or changing levels of salinity.

Corrections and further preparations for the measurement technique are required. Their instant use is therefore excluded from waters in this category which may be estuarine, or coastal, without substantial preparation of the sample. These factors are a significant drawback to their general use.

Other well known methods for analysis of ammonia/ammonium ion and other water contaminant species also exist using various 'wet' chemical techniques often relying upon a colour change or similar. These methods require even more of a degree of technical skill and experience to be used successfully than the ISE electrode technology discussed above.

Additionally these wet methods are prone to interference from the strong oxidising or reducing agents or complexing species found in the water samples. Well known wet methods require a sample to be taken to the laboratory for measurement with suitable bench-type equipment, e.g., ion chromatography, Kjeldahl apparatus, etc. However, these types of measurements are often accepted as standard, but require time, effort, and technical data interpretation by qualified staff.

Colour change chemistry methods such as the Nessler Method are also dependent upon the use of toxic mercury salts requiring additional measures to control safety and disposal of spent samples. The Nessler Method also suffers from a number of commonly found interfering species.

Thus to overcome these particular problems considerable time and effort is required for sample preparation and pre-treatment.

It is also generally the case that the ability of existing analysis measurement systems for ammonia/ ammonium ion and other target species to give data immediately or in-situ with ease and reliability in seawater and brackish waters is limited.

It is an object of the invention to provide an in-situ use sensor system and method that also mitigates some or all of the above disadvantages in relation to the monitoring of the concentration, or presence, or presence at a predetermined level of a target water contaminant and in particular a nutrient species such as ammonia/ammonium ion, nitrate, nitrite, etc. Preferred embodiments of the present invention seek to overcome the above described disadvantages of the prior art.

It is a particular object of the invention to provide a sensor system and method that employs established electrochemical principles in a manner that is reliable, cost effective and user friendly.

It is a particular object of the invention to provide a sensor system and method that lends itself to automatic use in in-situ site use in the field and/or operation by relatively untrained personal.

According to an aspect of the present invention there is provided a sensor system for assessing a target species in a liquid medium, said sensor system comprising a sensor element adapted to co-operate with a sample of liquid medium to be tested, said sensor element comprising at least three electrodes, and said sensor system further comprising a power source adapted to apply via a potentiostat a pre-determined potential to the sensor, said potential difference being determined by the potential associated with an electrochemical reaction characteristic in said liquid medium of said target species, and output means adapted to output data corresponding to the current passing through said liquid medium when a sample co-operates with said sensor element and said electrochemical reaction occurs.

According to another aspect of the present invention there is provided a method for assessing a target species in a liquid medium, comprising the steps of:
(i) introducing a sample of liquid medium to be tested to a sensor element adapted to co-operate with said sample, and said sensor element comprising at least three electrodes, the method further comprising
(ii) connecting said electrodes to a source of a potential difference related to a potential associated with an electrochemical reaction in said liquid medium characteristic of said target species; and
(iii) outputting data relating to the current flow between said electrodes caused by said potential of said characteristic reaction of said species.

Thus, according to the invention in a further aspect, a sensor system for measuring the concentration, or indicating the presence or presence at a predetermined level of, a target contaminant species in an aqueous medium comprises a sensor element having a sample receiving area for receiving a sample of aqueous medium to be sampled and which comprises at least three electrodes each comprising a layer of conductor deposited upon an insulating substrate, and further comprising a power source adapted to apply a pre-determined potential difference across two of the electrodes determined by the potential associated with an electrochemical reaction characteristic of the target species, and output means to output data corresponding to the current generated thereby when a sample is in place on the sampling area.

It is a particular object of the invention to provide a sensor system and method that can be applied to continuously operated monitoring applications and for extended periods of time.

It is a particular object of the invention to provide a sensor system and method then can provide for the measurement and analysis of nutrient species such as ammonia/ammonium ion, nitrate, nitrite, etc. in a concentration range covering sub ppm w/v to % w/v levels.

Thus according to the invention in an aspect, a sensor system for measuring the concentration, or indicating the presence or presence at a predetermined level a target contaminant species in an aqueous medium comprises a sensor element providing contact with a sample of aqueous medium and which comprises at least three electrodes each comprising of suitably and accurately dimensioned, high purity noble metal electrodes, and further comprising a power source adapted to apply a pre-determined potential difference across two of the electrodes determined by the potential associated with an electrochemical reaction characteristic of the target species, and output means to output data corresponding to the current generated thereby when a sample is in contact with the sampling area.

The active part of the sensor system thus comprises three or more active small mutually insulated electrode sites in the sample collection area and contacting the aqueous sample by being immersed in the sample solution. The general principles of chemistry underlying the operation of the sensor system in this active area will be known. The power source is connected to the sensor system to create a sensor system control circuit which can effectively function as apotentiostat.

Sensor system sensitivity performance can be improved by use of other well- known circuits producing chrono-amperometric, cyclic voltammetry measurements or better differential square wave pulsed techniques.

Two electrodes are polarised when immersed in the sample at a pre-determined potential characteristic of an electrochemical reaction (E°) indicative of the species under investigation. Polarising the electrodes results in generation of a current as the electrochemical reaction involving the target species proceeds, and this quite rapidly settles into a steady change state where the current is proportional to the concentration of the target species. One of the electrodes is used to provide a reference point so that the working electrode can be set at a specific potential. The third electrode, the counter, completes the circuit.

The applied voltage, which is carefully selected to carry out a specific and unique process or reaction in the sensor cell, results ultimately in the generation of an electric current. This current is directly related to the process being carried out at the sensor cell and is either an oxidation or reduction process at the working electrode. In one method of operation the sensor system concentration range can be extended by including a pre-measurement potential polarisation step. In this process the electrode is polarised at a potential close to the pre-determined potential characteristic of the electrochemical reaction. After a pre-determined and experimentally derived period of time allowing for the current value decay the specific reaction potential is applied and the measurement proceeds.

This 2-stage approach provides for measurement capability and over a significant range of concentration, but without the need for different electrode areas to be required.

Although the system employs known and established electrochemical principles, the sensor is greatly simplified over many prior art systems, which employ large-scale electrodes and/or which employ potentiometric principles where a potential difference is measured. In particular it will be seen, the sensor is in effect pre-calibrated and provided with an integral reference electrode. There is no need for calibration in the field.

The system in its portable configuration requires only a small sample of liquid to function effectively. For example a small volume of liquid to be tested in a receptacle in which the electrode is placed. Larger quantities of sample may also be used as in the continuously operated variant. For example the sensor is immersed into a liquid to be tested such that a sample contacts the sample receiving area, either by direct immersion in situ or, more conveniently, by immersion of the sensor in a quantity of collected sample in a suitable container.

The electrodes acting in this manner are small, cheap and in one such preferred embodiment are intended to be disposable. Dimensional control of the sensor element is tightly controlled, and the dimensions of the areas of the 3 or more electrodes are controlled to pre-determined tolerances. Further, the spacing between the 3 or more electrodes is also determined to set dimensional tolerances. Small variations in electrode surface areas can influence the measurement results and so is also controlled in the sensor unit manufacturing process. Because of the accurate control of properties of the electrodes which is possible by using conductor layer on insulating substrate technology, for example thick or thin film conductor technology, in particular with noble metals of very high purity, separate sensor electrode elements do not need to be separately calibrated. Purity of the metal deposited is crucial and highly important in obtaining acceptable repeatability.

For a given material and electrode configuration combination the behaviour of the electrodes varies to a sufficiently small extent, when using very pure metal, to render this unnecessary. Consequently the sensor system is self-referencing, and is therefore very easy to use in real environmental situations in the field to a reasonable level of accuracy.

A strong advantage of the present invention is in its ability to be used quickly for in-situ/in-field type measurements. In this respect an advantage by comparison with other methods that require collection of a sample and transport to a laboratory for analysis. Alternatively, pre-treating the sample on site and subsequent analysis at a laboratory is avoided the method provides for an immediacy of results.

In operation, a few millilitres, of solution is taken from the bulk sample, which might be a river, an ocean or simply a glass of water. This sample is placed for testing in a suitable receptacle.

In the continuously operated embodiment the electrodes are relatively small in size providing a low cost measurement solution.

In this embodiment, solution conditioning reagents are introduced to the sample by suitable valves and pumping system to deliver suitable and small quantities of the necessary reagents.

Further, the life-time of the sensor system in the continuous use embodiment can be extended by the periodic application of suitable potential changes to effectively and efficiently remove the collected or deposited reaction products from the electrode surface. The cleaning process is achieved by monitoring the current passed by the sensor and over time and when a pre-determined value is reached the cleaning process is actuated. This cleaning stage returns the electrode surface to an almost as new condition, allowing the sensor system to be operated remotely and for extended periods of time. This provides for a sensor system that allows a low cost of operation.

To the sample is added a pH buffering agent. This buffering agent is a preferably non-toxic chemical reagent, which will adjust the pH of the sample to a value acceptable and necessary for the characteristic electrochemical process to be tested. For example a buffering agent is selected to vary the pH of the initial solution to free into solution a species related to and indicative of the contaminant species under test, the characteristic electrochemical process to be tested by the sensor being one involving this related species.

In an example, when testing for ammonia/ammonium ion, a solution of interest would have a buffer added, changing the pH of the test solution to a pH value in excess of pH 9. At an alkaline pH value, and with the application of a suitable and specific voltage, free liberated ammonia present in the sample is oxidised, by the current producing electrochemical process.

In a sample where only ammonia is present the method provides for a method of detection and analysis where the solution pH varies between neutral and an alkaline value and using a cyclic voltammetry, or similar method with suitable peak detection algorithms used in the control system software. This approach could be used in applications where the ammonia is introduced as a gas, perhaps as a source of nutrient, and where it does not immediately convert to ammonium ions.

Additionally or alternatively to this sample may be added a standard reference reagent, that is a reagent which sets up a suitable standard reference cell in situ involving the reference electrode to provide a reference for the working electrode. For example, suitable for target species such as ammonia/ammonium ion, then sodium chloride is added to a suitable standard solution concentration, for example at a concentration of around 30 gl⁻¹ to the solution. That is part of the standard solution described which, in conjunction with the silver reference electrode, will set up a standard Ag/AgCl reference in situ on the device.

In all the measurement processes only a small volume of sample is required, hence only a small quantity of buffering reagent is required to adjust the sample pH to a desired level irrespective of the original pH of the sample solution source. Equally, only a small quantity of standard reference reagent is required to adjust concentration levels in the sample to reference levels and this is again largely irrespective of the original level in the sample solution source. Both of these might vary between, for example, seawater, drinking water, and treated sewage effluents. Hence the process is straightforward and achievable with little cost, and effective regardless of the sample solution source.

A number of advantages will be apparent over prior art systems.

First, the electrode units are inexpensive to manufacture, allowing a measurement to be made by the user at little cost. It is practical in one embodiment for the electrodes to be intended for disposable single use. The sensor is not manufactured from glass and is therefore less fragile. It is also practical in a second embodiment for the electrodes to be used continuously and over a long period of time.

Second, the sensor system in all configurations is simple to use. In a preferred embodiment it can be fabricated as a "black box" comprising, in compact operative association, a holder for a removable and optionally disposable electrode, a power source, an output means, access to the sample collection area when an electrode is inserted, and an actuator to initiate measurement, and optionally further a readily user readable display. Thus in a preferred embodiment a user merely needs to insert an electrode, place into the volume of sample, and press a button to initiate the measurement process. Hence there is no need or requirement for a highly trained or skilled operator.

In an alternative embodiment the process is equally simple. Conditioning reagent solutions are introduced via pipe-work to the sensor and the system is switched on. It then follows a pre-determined software based sequence of operations to facilitate the measurement process and data reporting.

Third, the measurement process is quick, requiring only one or two minutes or so before a subsequent measurement result is displayed. The measurement method is both safe and environmentally friendly, an important advantage over some existing methods. In practice the conditioning reagents do not require separate preparation (c.f. ISE system for example where a calibration is necessary then followed by reagent treatment of sample for measurement, etc,). With the sensor system all the necessary steps are obtained when the sample is put in contact with the electrode assembly.

An added and significant advance over the current methods of analysis generally available is that the sensor is largely unaffected by chemical materials commonly found in the aqueous environment. Debris will not influence the measurement process. Other ionic species present in the sample have been shown not to affect the sensor's performance. Deterioration of the sensor with time and routine use will not be a concern because of the intended protocols of operation. There is an ability to make measurements in aqueous samples that possess high levels of salinity. This will potentially allow measurements in estuarine waters, where salinity changes, or even in the sea and oceans and various industrial process streams where high levels of salinity are found. This is an important area where conventional ISE electrodes fail, or require special consideration of preparation. There are also areas in the medical world where an ammonia/ammonium ion sensor capable of use in a saline solution would be advantageous, e.g., blood, and urine analysis.

The power source causes the sensor system circuit to function as a potentiostat. To gain great sensitivity and repeatability, a variant of the simple potentiostatic circuit, that of a differential pulsed square wave voltametric circuit could be used, and suitable control means are provided in the power source to effect this. Alternatively, a simple chrono-amperometric method of operation could also be used, or another suitable electrochemical process.

The power source may be any suitable electrical power source, and is preferably portable for use in the field, and for example comprises a battery, for example remotely rechargeable, a fuel cell, optionally disposable, or the like.

The output means deals with a primary output of a quasi-steady state current, or alternatively a measured current value selected at a pre-determined region of potential and comprises processing means to output this as unit readable data corresponding to this current in any suitable form. For example, unit readable data may be suitable for output to any suitable display means and/or any suitable data register in data storage and/or processing means such as a computer. The output means might be adapted such that the data may correspond to measured current, which can then by compared to a reference database to produce useful data indicative of the concentration and/or presence and/or presence at a predetermined level of the target species in the solution under test.

Additionally or alternatively the output means may be associated with conversion circuitry to convert the primary output, for example by comparison to pre-recorded reference data, to secondary output comprising unit readable data directly indicative of indicative of the concentration and/or presence and/or presence at a predetermined level of the target species in the solution under test.

The sensor system preferably further comprises display means to display the output data in a user readable form. For example, the display may comprise an alphanumeric display indicative of the current generated in the circuit at steady state under test and/or indicative of the concentration and/or presence of the target species in the sample. Additionally or alternatively the display may comprise a digitised display adapted to indicate one of a small number of discrete states, for example presence or absence of the target species, or presence at a small number of pre-determined levels. Additionally or alternatively, the display may incorporate audio elements, for example emitting a sound if the target species is present or presence at a pre-determined level.

The sensor system also provides for efficient data transfer from a remote location to a laboratory base via internet or mobile phone data mechanisms.

The sensor electrode unit is formed as three or more conductor electrodes provided as layers of conductive material, for example thick or thin films, on an insulating substrate, and so configured that a sample is in contact with the three electrodes simultaneously. Each of the at least three electrodes therefore presents a free external surface comprising at least a part of the sample collection area.

For convenience and compactness it will generally be preferable that the at least three electrodes are deposited on a single supporting substrate.

In a preferred embodiment, at least some of the at least three electrodes are provided concentrically in the sample collection area. For example, in an embodiment where three electrodes are provided the electrodes comprise a first electrode making up a central generally circular portion, and second and third electrodes concentrically annular or partially annular there around. For example, the central electrodes can serve as the working electrodes, the first outer annular electrode can serve as the reference electrode, and the second outer annular electrode can serve as the counter electrode. Clearly other geometric designs are possible for the layout of the electrode assembly.

In an embodiment where four electrodes are provided, the electrodes may again be similarly concentrically arrayed. In an alternative embodiment, one or other of the outer annular electrode areas is divided into two mutually insulating portions, to comprise two of the four electrodes. Other geometries will readily suggest themselves. Close control over the dimensions of the electrode components is essential.

Each electrode comprises a layer of conducting material and in particular metallic material laid down upon an insulating substrate. Suitable layer thicknesses are preferably below 1 mm. The layer is preferably applied as a film by any suitable film technology to form a conductive film on the substrate surface. Metallic layers can be fabricated by any suitable method, such as for example screen-printing, sputtering, evaporation, and associated techniques which are known for laying down and bonding metals to hydrophobic substrates. Manufacturing techniques suitable for producing either thin film or thick film electrode units are suitable for the functioning of the sensor system.

Preferably, the electrodes are fabricated from noble metals of high purity, and in particular of materials selected from silver, gold, platinum, palladium in substantially pure form or as alloyed combinations thereof, in particular with additional impurity levels of less than 0.5%, more preferably less than 0.05% and more preferably still less than 0.01%.

In a second embodiment, and relevant to a continuously operated device, the sensor electrodes may be manufactured from accurately dimensioned noble bare metal substrates in intimate and direct contact with the sample medium, or positioned behind a permeable frit to provide for physical protection of the electrodes. Pre-calibration of the sensor electrodes in this embodiment is also a benefit of the sensor system. Electrodes fabricated from the materials and in the manner of the invention allow for carefully controlled electrode properties. As a consequence the system is in effect pre-calibrated, and it is not necessary to calibrate for each electrode. It is not necessary to calibrate the system in a reference solution before use in like manner to prior art sensor systems, in part because the invention enables the use of standard solution materials combined on the electrode units. The sensor system of the invention this offers the potential to replace more laborious laboratory based and less sensitive in-field analytical methods. Electrode materials and configurations can be pre-selected to be tailored to the electrochemical reaction to be used as characteristic of the target species.

Preferably, the sensor includes temperature measuring means and/or means to input a measured temperature at the time of sampling, and the conversion circuitry, or suitable software algorithms, or combinations thereof, then incorporates means to make a temperature compensation to raw output data based upon this temperature measurement relative to standard conditions.

The means to make a temperature compensation may comprise an electronic temperature compensation circuit. This temperature compensation circuit is used to overcome effects of external changes upon the standard and reference electrode potentials, and the temperature/current relationships via the Arrhenius Relationship. The temperature compensation circuitry becomes highly important when the system is used in hot countries or when there are temperature fluctuations. Alternatively to the temperature compensation circuitry would be an 'offset' controlled by an algorithm in suitable system software and related to a simple temperature measurement. Additionally or alternatively, the sensor includes conductivity measuring means to measure conductivity of the sample solution on the electrodes at the time of sampling, and the conversion circuitry incorporates means to make a conductivity compensation to raw output data based upon this measurement relative to standard conditions.

In practice there would be included provision to initiate a rapid and short measurement of electrical conductivity immediately after 'Switch On'. This short and rapid conductivity measurement would conveniently be coupled with the offset circuitry/ software described in relation to temperature correction and/or with offset circuitry/software analogous thereto. The stored values are compared or related to an independent knowledge of the value of conductivity of the standard solution. This provision would allow the equipment to be used without any external switch on the instrument when used for measurement of a wide variety of basic compositions (river-water, sea-water, etc).

The device thus always gives the same signal for the particular target species concentration being measured. Moreover, the occasional apparent curvature and minor variations noted of signal at low concentrations of target ion is amply corrected and eliminated.

The invention performs a simple test in a convenient and portable manner rapidly and without the need for detailed pre-calibration or for a separate reference electrode and is thus particularly suited to use in the field. The sensor is potentially more accurate than prior art systems for use in the field, and offers levels of accuracy to present an effective in field alternative to prior art use of sampling and subsequent examination by expensive laboratory equipment at a remote site.

Since the device measures changes in current, a direct and linear relationship exists to target species concentration, which further simplifies its use in determining such concentration. This contrasts with prior art potentiometric methods, where a logarithmic relationship exists between the potential change measured by the device and the concentration.

The electrode is self-referencing. In accordance with the principle of operation of the sensor system, this is achieved by provision of an integral reference electrode, and by chemical modification of the sample in situ to create a reference solution. This can be achieved by adding the reference medium to the sample in situ. However in a preferred embodiment, the sensor element is provided with a layer of a suitable chemical species deposited on the upper surface in the sample collection area such as to be rapidly and very soluble in the aqueous sample and placed thereon to effect formation of a suitable reference solution for operation of the reference electrode. Where applicable, a suitable buffering species may be similarly applied additionally or alternatively. In a preferred embodiment the addition of the conditioning reagents to the sample solution and application of a suitable potential will provide for the establishment of a suitable and stable reference electrode. For example, in many instances a silver/silver chloride reference system with an alkaline potassium or sodium chloride reference solution will be familiar. This is the case for instance in prior art systems for the measurements of ammonia/ammonium ion levels. In this instance, a sample is collected sodium chloride added to a standard level. This may be added separately once the sample is in place, but is conveniently provided in the form of a deposited layer of standard reagent to sufficient level to go into solution once the aqueous sample is placed on the sample collection area and thus effect formation of the referenced solution. Other deposited species will be appropriate for other reference standards.

A preferred method of manufacture of electrodes is to cover or paint the electrode area with standard mixture solution and allow to dry. The electrode is preferably then protectively packaged for environmental protection, for example protectively packed in plastic, say a thin polythene container for storage, before being used for measurements.

Alternatively, there is further provided a matting layer applicable to the electrode surface and preimpregnated with standard mixture solution. The matting may be very fine woven or non-woven material, such as plastic material, which has the capability to absorb water. This is soaked with standard mixture solution and allowed to dry. In a preferred embodiment, matting of suitable size and shape to cover the electrode contact area is provided in kit form in combination with an electrode to be slipped over or temporarily affixed on to the electrode prior to use. This would provide a larger reservoir than a simple painted surface preparation. Both alternatives avoid the need to provide additional standard reagent and offer a simple single use electrode element.

In accordance with the invention in a further aspect there is provided a method of measuring the concentration, or indicating the presence or presence at a predetermined level of a target contaminant species in an aqueous medium comprising the steps of:
placing a sensor element comprising at least three electrodes each comprising a layer of conductor deposited upon an insulating substrate into an aqueous sample to be tested;
optionally causing a suitable chemical species to go into solution in the aqueous sample to create a suitable reference solution for the pre-determined electrochemical reaction; connecting the electrode to a power source to set up a control circuit, for example a potentiostatic type or other suitable circuit, applying a pre- determined and suitable electrochemical process determined by the potential associated with an electrochemical reaction characteristic of the target species;
awaiting the establishment of a steady state or quasi steady state;
outputting data associated with the current of said steady state;
optionally converting the said output current data into data indicative of the
presence or presence at a pre-determined level and/or level of concentration of the target species in the sample;
and/or optionally displaying the output data or converted data on suitable user readable display means;
and/or optionally transmitting the output data or converted data to suitable data storage and/or processing means.

In one embodiment of the method a reference solution is created by addition of a suitable chemical species to the collected sample in situ in the sample collection area. In another embodiment the sample collection area of the sensor element is pre-prepared by provision of the suitable chemical species deposited thereon in solid form and able to go into solution when the aqueous sample is applied thereto to effect formation of the reference solution.

In a further embodiment the suitable chemical species are delivered by pumping from a suitable stock solution receptacle.

Other preferred features of the method of the invention will be understood by analogy with the foregoing.

The invention will now be described by way of example only with reference to Figures 1 to 6 of the accompanying drawings in which:
Figure 1 is a plan view of a three electrode sensor element in accordance with the invention, see also photograph Figure 1a;
Figure 2 is a schematic representation of the sensor element of Figure 1 incorporated into a potentiostatic circuit embodying the principles of the invention; and with reference to the use of the embodiment shown in the figures in analysing an example target species comprising ammonium;
Figure 3 is example circuitry for figure 2, shown in a simple first alternative in figure 3a, and in a modified alternative providing for temperature compensation in figure 3b;
Figure 4 is a graphical representation of the relationship of current to concentration for an example target species comprising ammonium;
Figure 5 is a plan view of an alternative four electrode sensor element suitable for Nitrate and Nitrite measurements and analysis in accordance with the invention;

In Figure 6 is a schematic side elevation showing a three electrode sensor element suitable for the continuously operated embodiment.

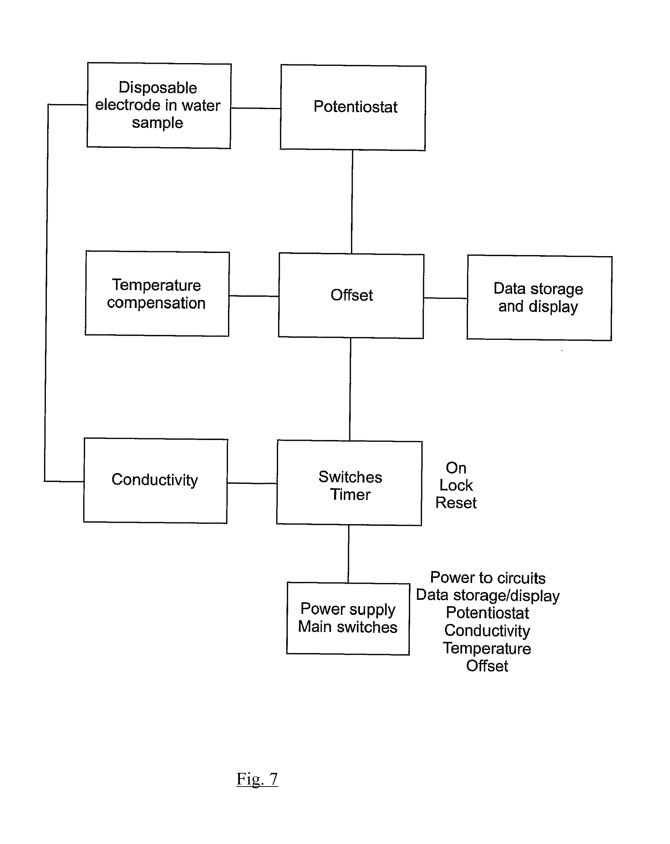

Figure 7 shows a possible comparator offset circuit arrangement to effect temperature and conductivity conversions.

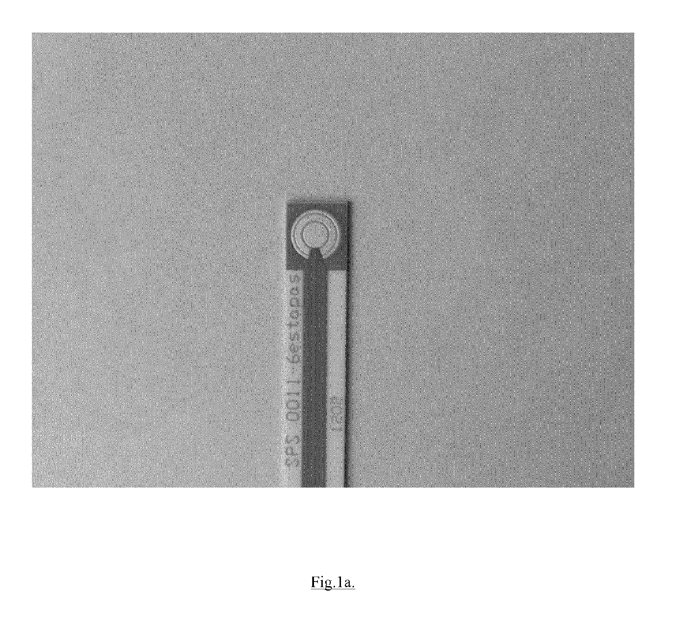

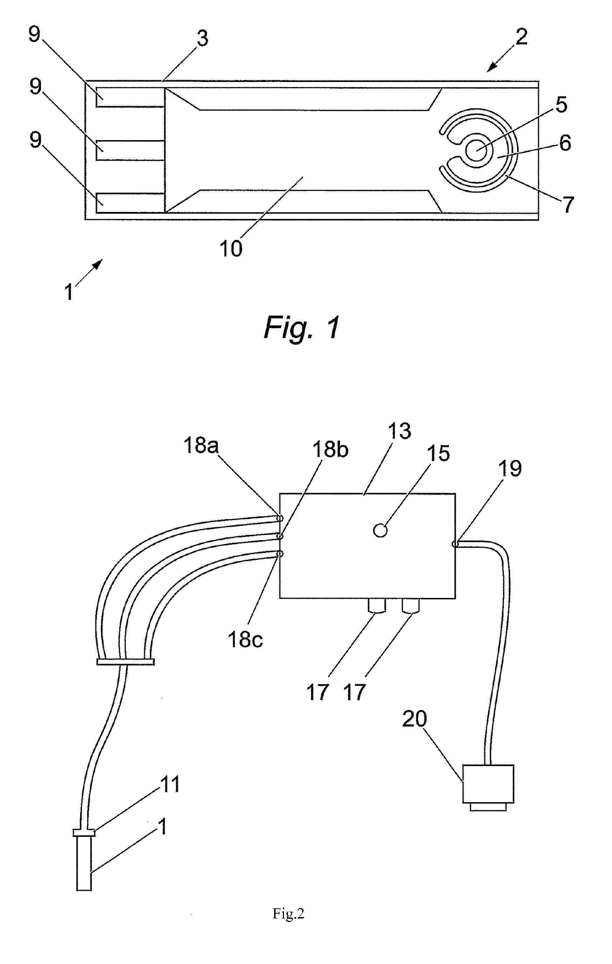

Referring to Figures 1 and 2, a three electrode model is shown suitable for analysing ammonia/ammonium ion. The sensor element (1) comprises noble metallic layers deposited upon an inert, hydrophobic and electrically insulating material, for example a corundum ceramic base (3). Deposited metallic layers comprise a working electrode (5) of silver, a reference electrode (6) of silver, and an auxiliary electrode (7) of silver. Three electrodes (5 to 7) are mutually insulated and connected to the other end of the sensor element by conducting paths (9). The electrodes are exposed in a small sample collection area (2), and the deposited conducting layers otherwise protected by a dielectric protection layer (10).

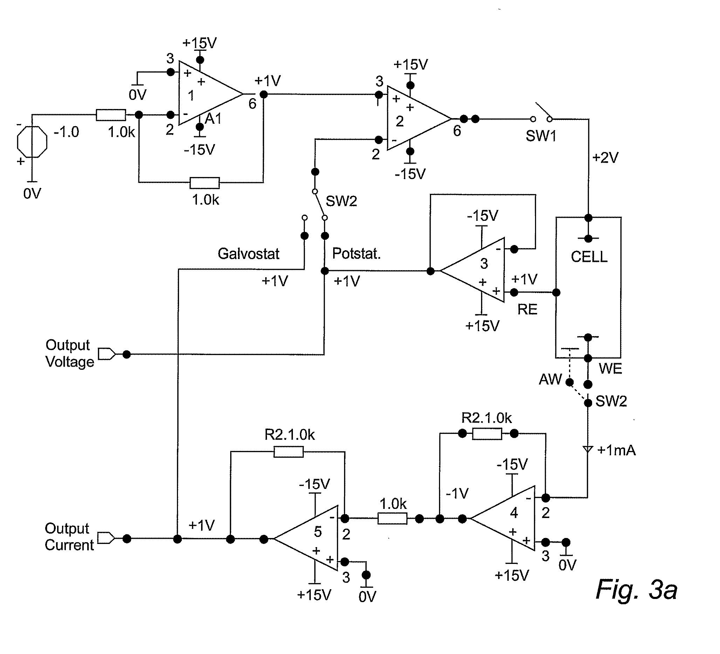

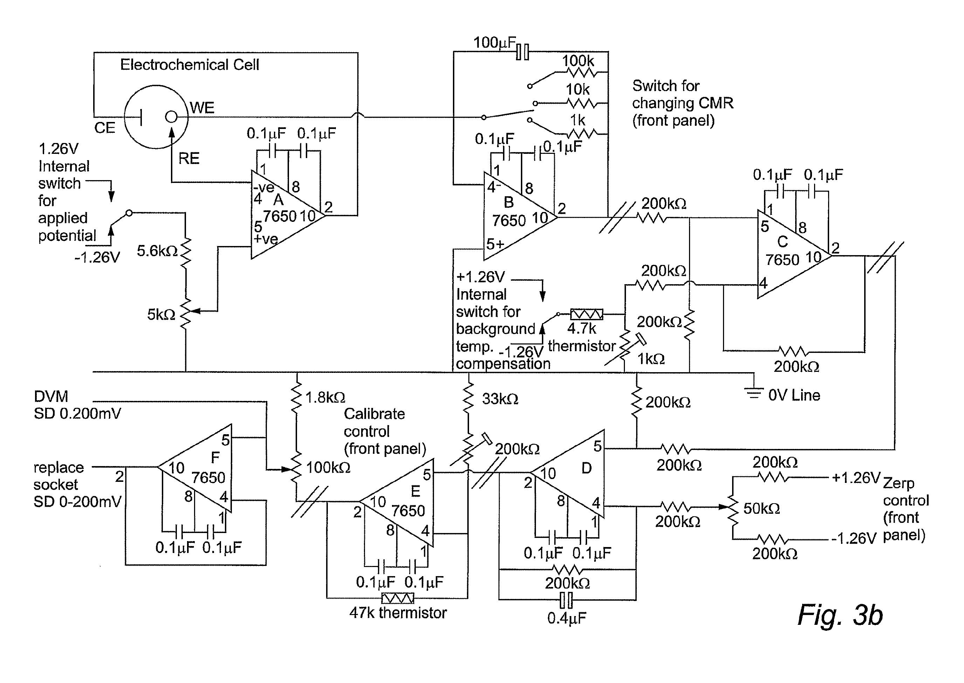

In use, a disposable electrode (1) is mounted in a housing (11) and a power source (13) drives the reaction. In the example, the measurement process is started by actuation of the button (15). Potentially static conditions can be varied by the control (17). Input data is collected from each electrode via the inputs (18 a-c), converted into machine readable form by the unit (13), and transmitted via the output (19) to a connection (20) for onward analysis by computer. A simple example of suitable circuitry is illustrated in Figure 3. Figure 3a is a basic circuit without provision for temperature compensation. Figure 3b incorporates two thermistors to connect for E⁰ variation with temperature and for the variation in signal current associated with changes in temperature.

In use testing for ammonia/ammonium ion the sensor element is placed/immersed in the aqueous sample (2). Already present is a reagent comprising a mixture of tri-sodium phosphate and sodium chloride.

The tri-sodium phosphate is a buffer reagent which will adjust the pH of the sample to a value, which is acceptable and necessary for the measurement process to be carried out. Our method relies upon changing the pH of the test solution to a pH value in excess of pH 9. At this alkaline pH value, and with the application of a suitable and specific voltage, free liberated ammonia present in the sample is oxidised. The degree of pH buffering is required to ensure a high degree of conversion of ammonium ion to ammonia irrespective of the original pH of the sample solution source.

The sodium chloride is a reference reagent. An Ag reference electrode is formed as soon as sample liquid (containing NaCl/Na₃PO₄) is present on the surface of the Ag substrate that is being used as the reference electrode. The concentration of NaCl is such that the conditions of the liquid test sample composition are considered. For example 30 gl⁻¹ NaCl would be suitable for pure water, drinking water, river water, seawater, and provides for a constant and the same reference electrode potential for all levels of chlorinity in the sample volume.

The use of standard solution in this manner has advantages in that: a) it provides chloride ions for the formation of a reliable reference electrode; and b) it allows the sensor system to be used for measurement in seawater, potable water, waste waters, drinking waters, etc.

It does not require additional treatments or reagents.

It provides a high pH, which ensures that the sample now contains free ammonia that is released from any ammonium ion present. It is then electrochemically oxidised by use of the potentiostat type circuitry, which furnishes a signal proportional to the concentration of ammonia or ammonium ion in the sample. No calibration of the system is required before or after use.

The relatively high concentration of chloride ions on Ag in the presence of water will immediately form AgCl on the surface. However in the presence NaCI at a concentration of 30 gl⁻¹ it closely approximates the conventional 'Standard' Reference (universally used) Ag, AgCl, 3.5 M KCl or thereabouts. The speed of this formation is very rapid; seconds, especially when compared with a prior art standard solution coated electrode.

Any variation of potential at these high concentrations of chloride ion is negligible, or can easily be corrected to the standard reference potential for comparison purposes. For example for variation of KCl concentration for standard reference by as much as 1M only effects its potential by as little 25 to 50 mV or less. Thus it is largely immaterial whether the initial sample was from seawater, freshwater, brackish sources, biological sources etc.

The suitable mixture of Na₃PO₄/NaCl, the standard reagent, may be dissolved directly into the liquid sample. A preferred method however is that during preparation of the electrode the reagent mixture dissolved in water is applied on the surface of the Ag electrode, dried and then sealed for subsequent use. Alternatively, a pre-soaked and dried piece of absorbent material (e.g., capillary matting, and like a stamp hinge) could be stuck to the surface. This item could be packaged similarly with the electrode itself.

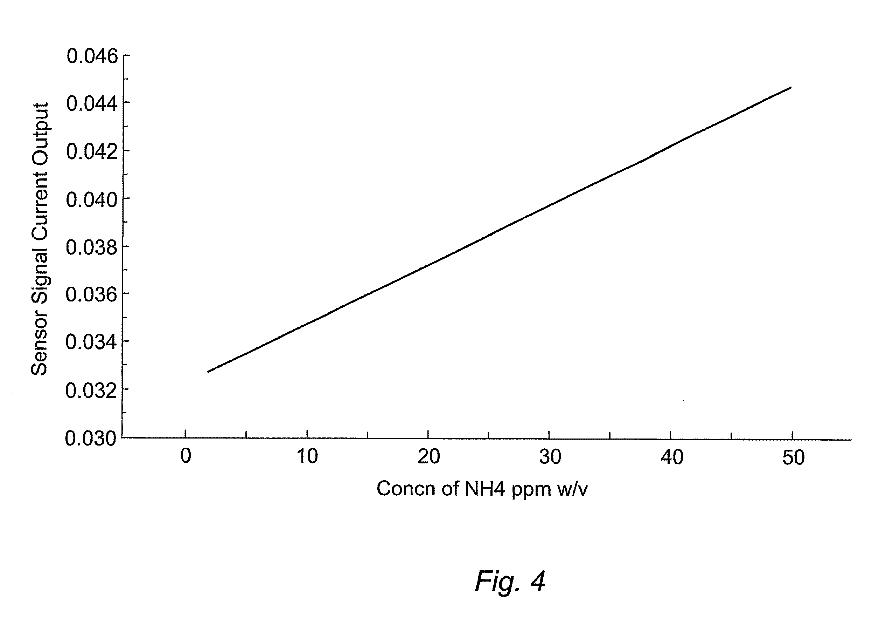

In use in the present example a sensor element (9) is immersed in the sample where the three working electrodes are exposed. The standard reagent effects change of the sample pH to a suitable alkaline pH value, and a specific suitable voltage applied to the working electrode (5) by the unit (13). The free liberated ammonia present in the alkaline sample is oxidised. The oxidation process is carried out electrochemically via the application of the specific and controlled voltage. The applied voltage is carefully selected to carry out the specific and unique process/reaction in the sensor cell, which results ultimately in the generation of electric current. This current is directly proportional to the concentration of the species being measured, and is related to the process being carried out in the sensor cell, as is illustrated for the working example ammonia/ammonium ion system by the plot of current versus concentration in figure 4.

The process described above in somewhat general terms can be explained further by a consideration of the principal electrode reactions. It is not suggested that the following reactions are definitive, or that the invention is in any way limited thereby, given the complex chemistry of silver and ammonia. However, as a suggestion for the reaction mechanism with a silver-working electrode the following could be indicated:

NH₃ + 90H⁻ - 8e → intermediate N0₃⁻ + 6H₂0 (Ag(NH₃)0H) complex

An electrochemical reduction now occurs simultaneously at the counter electrode:

8AgOH + 8e → 8Ag + 8OH⁻ (Ag₂O + H₂O → 2Ag(OH)) OH

Specificity is not considered a problem or limitation of the method for a number of reasons. The process is one of 'simply' oxidising dissolved ammonia electrochemically on silver and at around the oxidation/passivation potentials. Adsorption mechanisms are important in the process. Anions in solution cause no effect in the ammonia oxidation process at the working electrode.

Other background intermediate equations involving potentials, standard electrode potentials E° for the ammonia oxidation in alkaline solutions are given below. All of these identities of equilibria have almost identical values of E° which are known to those skilled in the art.
a) Ag₃PO₄ + 3e ↔ 3 Ag + PO₄³⁻
b) Ag(NH³)₂ + e ↔ Ag + NH₃
c) 2Ag + 20H⁻ ↔ 2AgOH + 2e
d) 2Ag + 20H⁻ ↔ Ag2O + H2O + 2e
e) Ag₂0 + H₂O + 2e ↔ 2Ag + 20H-

The sensor can provide measurements of ammonia and ammonium ions over the concentration range 0.5 to 100 ppm w/v. This can be extended to include up to several grams per litre levels of concentration by using the 2-stage potential step. Also, over a temperature range suitable for use in the field, with ambient temperature ranging from 5 to 30 °C at least.

Speed of response for the instrument to achieve 90 % of final value is around two minutes.

Minimum limit of detection 0.5 ppm w/v ammonium ion (perhaps less). The instrument allows measurements of species in saline solution up to, and including seawater strength.

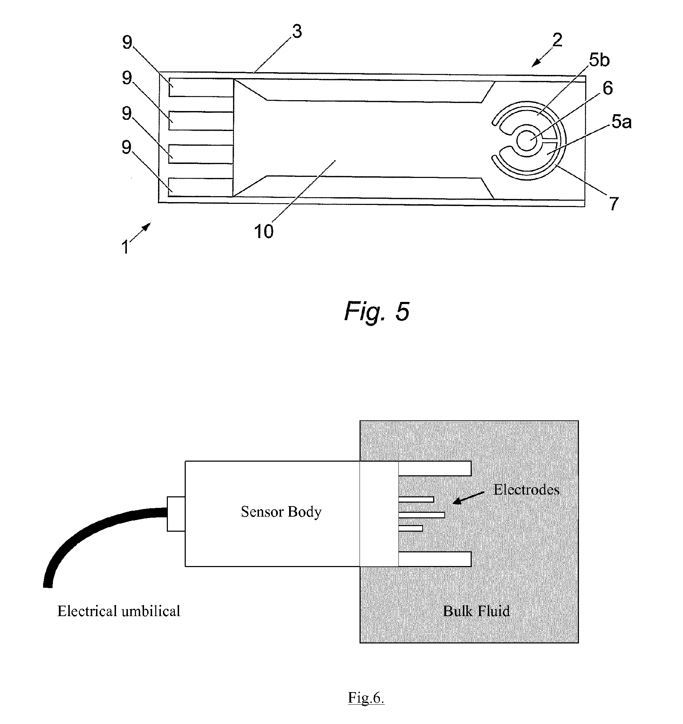

Figure 5 illustrates an alternative embodiment of sensor element (1). This is generally similar to the sensor element of Figure 1, and where applicable like reference numerals are used. However it is provided with four electrodes, specifically to deal with a nitrate based chemistry. In particular, two working electrodes (5a, 5b) are provided respectively to drive a reduction and an oxidation reaction. The four electrodes (5a, 5b, 6, 7) are mutually insulated as before and connected to the other end of the sensor element by four conducting paths (9). In some cases the fourth or a fifth electrode could be switched electronically to allow for electrical conductivity measurement corrections as described earlier, or generation of hydrogen as required.

The chemistry is as follows.

It is well known analytically, that nitrate (NO₃⁻) or nitrite (NO₂⁼) ions are easily reduced in alkaline solutions by the hydrogen that can be generated by the addition of metal Zinc.

In the present method however, hydrogen is not necessarily generated by application of a negative potential to the additional electrode (5b) in the sensor electrode assembly. All that may be necessary is the application of a potential of sufficient magnitude (the E° at pH 9 to 14) to automatically reduce the nitrate/nitrite ions to free ammonia. There are at least four electrodes. The illustrated example has four electrodes in total, the Working (5a), and Counter (6) and Reference (7) for measurement of the characteristic reaction, and the additional working electrode (5b) for reduction of nitrate/nitrite ion.

Two possible ways can be adopted for the reduction of nitrate or nitrite ion to provide free ammonia. The potential of electrode (5b) could be made sufficiently negative to generate hydrogen. Alternatively, and preferably hydrogen need not be generated, but by application of a potential around the E⁰ value for the reduction reaction involved for nitrate to ammonium ion (ammonia) i.e., a somewhat more positive value than that which is required to generate free hydrogen.

Moreover, the current derived from the conversion of nitrate/nitrite ion to ammonium ion, not employing hydrogen generation will also be directly related to their original concentration. In addition, by a further step change in potential it is possible to obtain the subsequent ammonia oxidation signal current.

Alternatively, a fourth electrode (5b) is not employed, but by a timer switch mechanism the working electrode is polarised to generate hydrogen for a short time. The potential is then changed to that required for the conversion of nitrite to ammonium ion.

Immediately a signal current is produced, under the control of the potentiostat, due to the oxidation of the NH₃ that has been formed earlier. The signal current is linearly and directly proportional to the concentration of NO₃⁻/NO₂⁼ present in the test sample being measured. A final output signal, on the basis given for the previously mentioned NH₄/NH₃⁺ sensor system, is generated after about two minutes.

From the above it will be realised that the total NO₃⁻/NO₂⁼ concentration of ions is measured in the test sample. Because of the close chemical relationship between NO₃⁻ and NO₂⁼ in practical terms this total quantity is the most important environmentally. However, should a measurement of either one or the other be required, when either or both are present, an alternative test based on an alternative characteristics reaction and/or an alternative form of the 4-electrode system may be employed.

In the present example in the preparation of the electrode assembly the substrate is coated as described earlier for the NH₄/NH₃ type but now with a suitable mixture of containing a reagent to provide for a pH of around 6.8. In this case at the suitable potential for only the NO₃⁼ ion concentration would be obtained. Clearly, two separate disposable electrodes would be used for measurements. Thus, would be made one giving the total and the other only NO₃⁼, simple subtraction could provide a value for NO₂⁼ ion concentration.

Other geometries of electrodes and preparations will be appropriate to other characteristic reactions for other target species.

Figure 6 shows a possible comparator offset circuit arrangement to effect temperature and conductivity conversions to raw data to accommodate deviation from standard conditions.

The circuit helps to correct the signal produced by the potentiostat before it passes into storage/display etc. The potentiostat current signal from the electrochemical process (oxidation of ammonia in this case) and as generally preferred is converted into a voltage output signal by the usual means. The value is fed into the offset on one of the three channels provided: the other two channel inputs are for the conductivity circuit (when on 'lock' status as below) and the temperature compensation circuit, also in voltage modes. A net voltage potentiostat signal due to positive and negative input voltages from the conductivity and temperature compensation is delivered. This resultant signal is then fed into the store/display part of the system.

The conductivity circuit in the example is a conventional and simple high frequency ac type circuit. It is connected to the fourth electrode on the disposable electrode assembly and the counter electrode. A conductivity measurement is made and after three of four seconds the circuit output is electronically 'locked' to provide a constant low dc output to one of the three inputs on the offset circuit.

The following description reiterates and supplements the description set out above. A sensor system is provided for determining the presence, and if required concentration, of a target species in a liquid medium. This could, for example, the detection of a contaminant in an aqueous medium such as ammonia in drinking water, river water or effluent discharge. The sensor system includes a sensor element 1 that is adapted to co-operate with and be inserted into the liquid medium that is being tested. The sensor element 1 has at least three electrodes 5, 6 and 7 and one of these electrodes is preferably used as a reference electrode. The sensor system also includes a power source 13 which applies a predetermined potential, via a potentiostat to the sensor. The potential is determined by the potential associated with an electric chemical reaction characteristic in the liquid medium of the target species. The sensor system further includes an output, in the form of connector 20, that output data corresponding to the current flowing through the liquid medium when the sensor element 1 is inserted into the sample and the electrochemical reaction is occurring due to the potential applied across the electrodes of the sensor.

The sensor element 1 is removable and replaceable within a housing 11. Each sensor element 1 includes an insulating substrate, in the form of a ceramic base 3, onto which a layer of conductor is deposited. In order for the sensor element 1 to produce consistent readings from one replaceable sensor element to the next, the electrodes must be consistently and accurately dimensioned on the substrate and are preferably formed from a high purity noble metal. These noble metals are preferably selected from silver, gold, platinum and palladium and are in substantially pure form with impurity levels of less than 0.5%, more preferably less than 0.05% and most preferably less than 0.01%. However, non-metallic electrodes can be used. Bare metal electrodes can also be used, particularly where the apparatus is used for continuous monitoring, wherein variations in contaminant level are the target rather than absolute values of the contaminant. In this continuous monitoring, which is typically within a flowing aqueous body. A chemical species is provided to effect the formulation of a reference solution and/or buffering of the aqueous body adjacent the electrode assembly. In this situation the power source and potentiostat circuit are adapted to provide a continuous and long-term operation in a remote environment.

Prior to the insertion of the sensor element 1 into the sample, the sample has a pH buffering agent added thereto to adjust the pH of the sample to a preferred value which is characteristic of the electrochemical reaction. Examples of these pH buffering agents and the characteristic electric chemical reactions are set out above. This variation of the pH by the addition of the buffering agent force into solution a species related to and indicative of the target species which the sensor is determining the presence and concentration of and being the species related to the characteristic electric current reaction performed by the sensor within the sample.

The sensor system may also include the use of a reference agent (for example Sodium Chloride) which is added to the sample of liquid medium when sensor element is introduced into the sample. This reference agent as well as the buffering solution may be deposited directly onto the sensor element, preferably on the electrode area in the form of a dry layer or as a matting layer.

The power source can be integrated into a circuit with the sensor element so that the sensor element is caused to function as the potentiostat. The power source may also include a controller which acts in combination with the sensor element to create one of a range of electric coprocessors which affects the assessment of the target species. The controller can also co-operate with the sensor element in an electric chemical process that provides a two-stage application of the potential difference applied to the sensor element.

Where the apparatus is used for continuous or repeated measurements in a flow of liquid medium, a measure of the current passing through the liquid medium over time is used to determine the condition of the sensor and, if appropriate, to initiate a change in the potential applied to the electrode to facilitate cleaning of the electrode surface.

The output of the sensor system is connected to a display device which includes an alphanumeric display providing an indication of the current passing through the liquid medium at steady-state under test and/or that is indicative of the concentration or presence of the target species in the sample of liquid medium. This may alternatively be a digitised display that indicates one of a small number of discrete states including, but not limited to, the presence or absence of the target species or presence at a small number of predetermined levels.

The sensor element 1 preferably includes three electrodes, 5, 6 and 7 and these are arranged concentrically with respect to each other and most preferably, as shown in figure 1, with a central first circular electrode 5 and two annular or partially annular electrodes 6 and 7 arrange concentrically around the circular electrode 5. In a variation on this formation one of the annular or partially annular electrodes may be divided into two portions, the sensor element therefore comprising four electrodes.

The sensor element 1 may include temperature measuring apparatus to allow for compensation for variations in temperature. It may also include conductivity measuring means to allow the output to compensate for variations in conductivity within the sample.

It will be appreciated by persons skilled in the art that the above embodiments have been described by way of example only and not in any limitative sense, and that various alterations and modifications are possible without departure from the scope of the protection which is defined by the appended claims.

## Claims

1. A sensor system for assessing a target species in a liquid medium, said sensor system comprising a sensor element adapted to co-operate with a sample of liquid medium to be tested, said sensor element comprising at least three electrodes, and said sensor system further comprising a power source adapted to apply via a potentiostat a pre-determined potential to the sensor, said potential being determined by the potential associated with an electrochemical reaction characteristic in said liquid medium of said target species, and output means adapted to output data corresponding to the current passing through said liquid medium when a sample co-operates with said sensor element and said electrochemical reaction occurs.

2. A sensor system according to claim 1 further comprising one or more of the following features:-
a) wherein said assessment effected by said sensor system is adapted for measuring the concentration, or indicating the presence or presence at a predetermined concentration of said target species;
b) wherein said liquid medium is an aqueous medium, and said target species is a contaminant therein;
c) adapted for the step of exchanging said sensor element for a replacement such sensor element between successive assessments of said target species in said liquid medium, said electrodes of said sensor elements each comprising a layer of conductor deposited upon an insulating substrate;
d) a pH buffering agent to be combined with said sample of liquid medium when it is placed for co-operation with said sensor element, said pH buffering agent being adapted to adjust the pH of the sample to a preferred value for said characteristic electrochemical reaction;
e) a reference reagent to be combined with said sample of said liquid medium when it is placed for co-operation with said sensor element, to set up a reference cell in situ and wherein one of the electrodes of said sensor element constitutes a reference electrode to provide a reference for the other electrodes;
f) wherein said power source and potentiostat circuit is adapted to provide for continuous and long term operation in a remote environment;
g) wherein said power source is integrated into a circuit with the sensor element such said sensor element is caused to function as a potentiostat;
h) wherein means to provide a measure of said current passing through said liquid medium over time is used to determine the condition of said sensor element and to initiate a change in potential applied thereto that will allow cleaning of the electrode surface;
i) wherein said power source is a portable electrical power supply for use in the field;
j) display means to display said output data in a user readable form;
k) wherein said sensor element comprises at least three electrodes deposited on a single supporting substrate with clearly and accurately defined dimensional tolerances;
l) wherein the electrodes comprise thin films of conducting metallic material laid down upon an insulating substrate;
m) wherein the electrodes comprise noble metals selected from silver, gold, platinum, palladium in substantially pure form or as alloyed combinations thereof;
n) wherein the sensor includes temperature measuring means and/or means to input a measured temperature at the time of sampling, and further comprises means to make a temperature compensation to raw output data based upon this temperature measurement relative to standard conditions; and
o) wherein the sensor includes conductivity measuring means to measure solution conductivity, and further comprises means to make a compensation to raw output data based upon this measurement relative to standard conditions.

3. A sensor system in accordance with claim 2 further comprising one or more of the following features:-
a) wherein said buffering agent is adapted to vary the pH of said sample of liquid medium to free into solution a species related to and indicative of said target species, said characteristic electrochemical reaction involving this related species;
b) wherein said reference electrode comprises a layer of a chemical species deposited on a surface thereof and adapted to be rapidly soluble in said liquid medium so as to effect formation of the said reference solution and/or buffering solution;
c) wherein said power source comprises control means adapted to act in combination with said sensor element to create one of a range of electrochemical processes to effect said assessment of said target species;
d) wherein said power source comprises control means adapted to co-operate with said sensor element in an electrochemical process that provides a 2-stage application of potential thereto;
e) wherein said display means comprises an alphanumeric display indicative of the current passing through said liquid medium at steady state under test and/or indicative of the concentration and/or presence of said target species in said sample of said liquid medium;
f) wherein said display means comprises a digitised display adapted to indicate one of a small number of discrete states, for example presence or absence of the target species, or presence at a small number of pre-determined levels;
g) wherein at least some of said at least three electrodes are provided concentrically with respect to each other; and
h) wherein the said noble metals or alloys thereof have additional impurity levels of less than 0.5%, more preferably less than 0.05% and more preferably still less than 0.01%.

4. A sensor system according to claim 3 further comprising one or more of the following features:-
a) wherein said chemical species is deposited directly on the electrode area of said sensor element so as to form a dry layer thereon;
b) a matting layer applied to the surface of at least one of said electrodes during use and preimpregnated with said chemical species;
c) wherein said electrodes comprise a first electrode providing a central generally circular electrode portion, and second and third electrodes disposed concentrically annular or partially annular with respect thereto; and
d) wherein four electrodes are provided and the electrodes comprise a first electrode making up a central generally circular portion, and second and third annular electrode portions disposed concentrically, or annularly, or partially annularly there around, wherein, one or other of the outer annular electrode areas is divided into two mutually insulating portions, to comprise two of the four electrodes.

5. A sensor system according to claim 1 or 2 wherein said electrodes comprise a bare metal electrode assembly, or non-metallic electrodes, or a combination of electrode components of accurately dimensioned and high purity noble metal components.

6. A sensor system in accordance with Claim 5 adapted for placement in a flowing aqueous body and comprising a chemical species to effect the formation of a reference solution and/or buffering of said aqueous body local to the electrode assembly.

7. A method for assessing a target species in a liquid medium, comprising the steps of:
(i) introducing a sample of liquid medium to be tested to a sensor element adapted to co-operate with said sample, and said sensor element comprising at least three electrodes, the method further comprising
(ii) connecting said electrodes to a source of a potential difference related to a potential associated with an electrochemical reaction in said liquid medium characteristic of said target species; and
(iii) outputting data relating to the current flow between said electrodes caused by said potential of said characteristic reaction of said species.

8. A method according to claim 7 further comprising one or more of the following features:-
a) wherein said liquid medium is an aqueous medium, and said target species is a contaminant therein;
b) the step of awaiting the establishment of a steady state or quasi steady state relating to said characteristic reaction before or in relation to said step of outputting said data relating to said current flow;
c) the step of exchanging said sensor element for a replacement such sensor element between successive assessments of said target species in said liquid medium, said electrodes of said sensor element each comprising a layer of conductor deposited upon an insulating substrate;
d) the further steps of converting the said output current data into data indicative of the presence or presence at a pre-determined level and/or level of concentration of the target species in the sample; and/or displaying the output data or converted data on suitable user readable display means; and/or transmitting the output data or converted data to suitable data storage and/or processing means;
e) the further step of causing a suitable chemical species to go into solution in said liquid medium to be tested to create a suitable reference solution for said electrochemical reaction;
f) the further steps of introducing a suitable chemical species to enter into said liquid medium to be tested to create a suitable reference solution for the pre-determined electrochemical reaction; and
g) the further steps of causing a suitable buffering solution to enter into said liquid medium to be tested to adjust the pH of the sample to a preferred value for said electrochemical reaction.

9. A method according to claim 8 further comprising one or more of the following features:-
a) the further step of causing a suitable buffering agent to go into solution in said liquid medium to be tested to adjust the pH of the sample to a value preferred for said electrochemical reaction;
b) wherein the said reference or buffer reference solution is created by addition of a suitable chemical species to the collected sample of liquid medium to be tested; and
c) wherein the said buffering solution is introduced by the addition of a chemical species stock solution.

10. A method according to claim 9 wherein said chemical species deposited on said electrodes in solid form and adapted to go into solution when the liquid sample is applied thereto to effect formation of the reference solution.

11. For use in a sensor system for measuring the concentration, or indicating the presence or presence at a predetermined concentration of, a target species in a liquid medium, a sensor element adapted to co-operate with a sample of liquid medium to be determined, said sensor element comprising at least three electrodes and adapted to be connected to a power source capable of applying a pre-determined potential to said sensor, said potential being determined by the potential associated with an electrochemical reaction characteristic in said liquid medium of said target species, and said sensor element being further adapted to be connected to output means for outputting data corresponding to the current passing through said liquid medium when a sample co-operates with said sensor element and said electrochemical reaction occurs.

12. A sensor according to claim 11 further comprising one or more of the following features:-
a) wherein said senor is adapted for uses wherein said liquid medium is an aqueous medium, and said target species is a contaminant therein;
b) wherein said sensor element is adapted to be replaced between successive assessments of said target species in said liquid medium, said electrodes of said sensor element each comprising a layer of conductor deposited upon an insulating substrate; and
c) wherein said sensor element comprises an accurately dimensioned noble metal electrode assembly.
